# EUROPEAN PATENT APPLICATION

(11) **EP 3 692 978 A1**
(43) Date of publication of application: **12.08.2020**
(21) Application number: 19155910.3
(22) Date of filing: 07.02.2019
(51) Int. Cl.: A61K 8/64, A61Q 7/02

(54) **NOVEL METHOD**

(71) Applicant: DSM IP Assets B.V., 6411 TE Heerlen (NL)
(72) Inventor: The designation of the inventor has not yet been filed
(74) Representative: Berg, Katja

(57) **Abstract**

The invention relates to a method for reducing hair growth in humans, particularly for cosmetic purposes.

## Description

The invention relates to a method for reducing hair growth in humans, particularly for cosmetic purposes.

Hair is a characteristic feature of all mammals, including humans. It is primarily composed of proteins, in particular keratin, and is produced in hair follicles within the skin.

There are many reasons, including cultural, cosmetic, or medical reasons, why individuals wish to remove some or all hair from sites of their body. Thus, already for decades, various procedures have been employed to remove unwanted hair, most of them being based on depilation (removal of the part of the hair above the surface of the skin) or epilation (removal of the entire hair). All procedures, however, have significant drawbacks associated with them. Shaving, for example, may cause skin abrasion or cuts, and can lead to a perception of roughness caused by sharp, stubbly hairs as the hair reappears after shaving. Depilation e.g. by waxing or trimming on the other hand is an intrinsically painful and uncomfortable process. Depilatory creams, even though being highly effective, are often messy and difficult to apply and can cause severe skin irritations. Antiandrogens, which are used to treat female hirsutism, reportedly can have significant side effects, mainly due to their effect on the hormone balance in the body.

Thus, there is an ongoing need for efficient and safe compounds which inhibit, reduce or delay the (re-)growth of hair, which compounds can e.g. be used in adjunction with epilation or depilation to prolong the time interval between the mechanical or chemical treatment.

Surprisingly, it has now been found that compounds of formula (I) as depicted below respectively a salt thereof are highly efficient in the inhibition of the growth of hair.

Thus, in a first aspect, the invention relates to a cosmetic or a therapeutic method (typically a cosmetic method) of reducing hair growth, said method comprising selecting an area of skin from which reduced hair growth is desired and applying to said area a topical composition comprising at least one compound of formula (I) 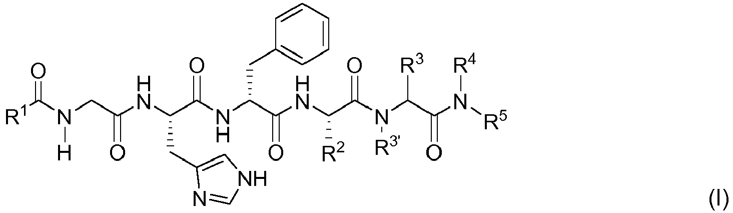 wherein
R¹ is selected from the group consisting of a C₁-C₁₀ alkyl group, a C₃-C₆ cycloalkyl group, an aryl group or an aryl C₁-C₆ alkyl group;
R² is an amino acid side chain of a basic amino acid or an amino acid side chain of a 2-amino C₂₋₈ alkanoic acid;
R³ and R^{3'} are selected from the group consisting of H, an arylC₁-C₆ alkyl group or a heteroarylC₁-C₆ alkyl group, wherein the aryl respectively the heteroaryl can optionally be substituted,
R⁴ and R⁵ are, independently of each other, H or a C₁-C₁₀ alkyl group; and
with the proviso that only one of R³ or R^{3'} is H and the respective other one of R³ and R^{3'} is not H
or a salt thereof in an amount effective to reduce hair growth.

The unwanted hair growth may be normal but undesirable from a cosmetic perspective or may result, for example, from the symptoms of a disease or an abnormal condition such as hirsutism or hypertrichosis. Individuals who have hair bearing naevi or pseudofolliculitis barbae may also wish to achieve a reduction of hair growth according to the present invention.

In a second aspect, the invention relates to the use of a compound of formula (I) or a salt thereof for the inhibition of hair growth in an individual in need thereof.

In further aspect, the invention provides a method (typically a cosmetic (non-therapeutic) method) of reducing unwanted hair growth by applying to the skin a compound of formula (I) or a salt thereof in an amount effective to reduce hair growth.

In addition, the present invention relates to a compound of formula (I) or a salt thereof respectively a topical composition comprising said compound or the salt thereof for reducing hair growth in individuals suffering from a disorder resulting in excessive hair growth such as hirsutism or hypertrichosis.

The term 'Cₓ-C_{y}alkyl group' as used herein refers to unbranched Cₓ-C_{y}alkyl or branched C₃-C_{y}alkyl groups such as methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and 3,5,5-trimethylhexyl groups.

The term 'C₃-C₆cycloalkyl group' as used herein refers to a saturated, 3 to 6 membered hydrocarbon ring such as cyclopropyl, cyclobutyl, cyclopentyl and cyclohexyl. Preferably in all embodiments of the present invention the C₃-C₆cycloalkyl group is cyclopropyl, cyclopentyl or cyclohexyl, most preferably cyclohexyl.

The term "aryl" as used herein refers to an aromatic substituent containing 5 to 15 carbon atoms and containing a single aromatic ring or multiple aromatic rings which are fused together, directly linked or indirectly linked (such that the different aromatic rings are bound to a common group such as a methylene or ethylene group). Particularly advantageous aryl groups according to the present invention contain 6 to 12 carbon atoms containing a single aromatic ring or multiple aromatic rings which are fused together or directly linked. Most preferred aryl residues in all embodiments of the present invention are phenyl, naphtyl and biphenyl.

The term "side chain" of an amino acid as used herein refers to that portion of the amino acid attached to the common 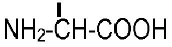 backbone of the respective amino acids. For instance, the side chain of serine is -CH₂-OH and the side chain of alanine is -CH₃.

The term "basic amino acid" as used herein refers to any natural or unnatural amino acid that have basic side chains at neutral pH such as the natural occurring amino acids arginine (Arg), lysine (Lys), and histidine (His) as well as the unnatural amino acids 2,4-diaminobutyric acid, homolysine and ornithine without being limited thereto. Advantageous amino acid side in all embodiments of the present invention are the side chains of arginine, lysine, 2,4-diaminobutyric acid, homolysine and ornithine such as in particular the side chains of arginine and 2,4-diaminobutyaric acid.

The term "2-amino C₂₋₈ alkanoic acid" as used herein refers to amino acids having a C₂₋₈ alkyl side chain, preferably a linear C₄₋₇ alkyl side chain. Most preferred 2-amino C₂₋₈ alkanoic acids in all embodiments of the present invention are 2-amino butanoic acid, 2-amino pentanoic acid, 2-amino hexanoic acid or 2-amino heptanoic acid such as in particular 2-amino hexanoic acid.

The term "arylC₁-C₆ alkyl group" as used herein refers to a -C₁-C₆ alkyl-aryl group, wherein the term "aryl" is as defined above. Advantageous arylC₁-C₆ alkyl groups are arylC₁-C₂ alkyl groups such as in particular phenyl(m)ethyl or naphthyl(m)ethyl.

The term 'heteroarylC₁-C₆ alkyl group' as used herein refers to a -C₁-C₆ alkyl-heteroaryl wherein the term "heteroaryl" refers to a 5- or 6-membered aromatic ring containing one or more heteroatoms, viz., N, O or S; these heteroaromatic rings may be fused to other aromatic systems. Particularly preferred heteroaromatic rings encompass indole, pyridine and quinoline. In all embodiments of the present invention preferred heteroarylC₁-C₆ alkyl group are heteroarylC₁-C₂ alkyl groups such as (1H-indol-3-yl)(m)ethyl, (pyridin-2-yl)(m)ethyl, (pyridin-3-yl)(m)ethyl, (quinolin-2-yl)(m)ethyl and (quinolin-3-yl)(m)ethyl groups.

The aromatic aryl respectively heteroaryl residues may, independently of each other, be unsubstituted or substituted with one or more substituents. In all embodiments of the present invention, such substituents are preferably selected from halogen, hydroxy, nitro, cyano, C₁-C₆ alkyl, C₁-C₆ alkoxy and C₁-C₆ alkanoyloxy. More preferably in all embodiments of the present invention the aryl respectively heteroaryl residues are, independently of each other, unsubstituted or substituted with one substituent selected from the group consisting of F, Cl, hydroxy, cyano, C₁-C₃ alkyl, C₁-C₃ alkoxy and C₁-C₃ alkanoyloxy such as in particular unsubstituted or substituted with one substituent selected from the group consisting of F or hydroxy. Most preferably, in all embodiments of the present invention, the aryl and the heteroaryl residues are unsubstituted.

In all embodiments of the present invention particular preferred heteroarylC₁-C₂ alkyl groups are (1H-indol-3-yl)(m)ethyl, 5-fluoro(1H-indol-3-yl)(m)ethyl, 6-fluoro(1H-indol-3-yl)(m)ethyl, 5-hydroxy(1H-indol-3-yl)(m)ethyl, (pyridin-2-yl)(m)ethyl, (pyridin-3-yl)(m)ethyl, (quinolin-2-yl)(m)ethyl and (quinolin-3-yl)(m)ethyl. Most preferred in all embodiments of the present invention are the heteroarylC₁ alkyl groups (1H-indol-3-yl)methyl, 5-fluoro(1H-indol-3-yl)methyl, 6-fluoro(1H-indol-3-yl)8m)ethyl, 5-hydroxy(1H-indol-3-yl)methyl, (pyridin-2-yl)methyl, (pyridin-3-yl)methyl, (quinolin-2-yl)methyl and (quinolin-3-yl)methyl and the heteroarylC₂ alkyl groups (1H-indol-3-yl)ethyl such as in particular (1H-indol-3-yl)methyl and (1H-indol-3-yl)ethyl.

The term 'or a salt thereof' refers to all conventional non-toxic salts of compounds of formula (I), such as those formed from organic or mineral acids, such as for example the respective acetates, trifluoro acetates, mesylates, citrates, oleates, oxalates or gluconates as well as chlorides, sulfates, borates or carbonates. The nature of the salt is not critical, provided that it is cosmetically, dermatologically and/ or pharmaceutically acceptable. Such salts are well known to a person skilled in the art. The cosmetically, dermatologically or pharmaceutically acceptable salts of the compounds of formula (I) can be obtained by conventional methods that are well known in the state of the art.

Most preferred in all embodiments of the present invention are the compounds of formula (I) as such or in the form of their acetates, trifluoroacetates (i.e. as 2,2,2-trifluoroacetates) or mesylates. Such salts are easily prepared by a person skilled in the art.

It is well understood, that the present invention encompasses the compounds of formula (I) as optically pure isomers such as e.g. as pure enantiomers or stereoisomers as well as mixtures of different isomers such as e.g. as racemates, or mixtures of diastereoisomers.

In all embodiments according to the present invention it is preferred that
- R³ and R^{3'} are selected from the group consisting of H, an unsubstituted aryl(m)ethyl group and an unsubstituted heteroaryl(m)ethyl group, most preferably R³ is selected from the group consisting of H, phenylmethyl, naphthylmethyl and (1H-indol-3-yl)methyl and R^{3'} is H or (1H-indol-3-yl)ethyl;
- R⁴ and R⁵ are, independently of each other, H or a C₃-C₈ alkyl group, most preferably H, propyl or octyl; and
with the proviso that only one of R³ or R^{3'} is H and the respective other one of R³ and R^{3'} is not H (but the respective arylC₁-C₆ alkyl group or heteroarylC₁-C₆ alkyl group, with all the definitions and preferences as given herein for R³ and R^{3'})

Particularly preferred compounds of formula (I) respectively a salt thereof such as in particular the acetate, trifluoroacetate or mesylate salt thereof in all embodiments of the present invention, are compounds of formula (I) wherein
R¹ is a C₁-C₂ alkyl group or a C₆-C₁₂ aryl group;
R² is an amino acid side chain of a basic amino acid;
R³ and R^{3'} are selected from the group consisting of H, an unsubstituted aryl(m)ethyl group or an unsubstituted heteroaryl(m)ethyl group; and
R⁴ and R⁵ are, independently of each other H or a C₁-C₁₀alkyl group; and
with the proviso that only one of R³ or R^{3'} is H and the respective other one of R³ and R^{3'} is not H.

Even more preferred compounds of formula (I) or salts thereof such as in particular the trifluoroacetate or mesylate salt thereof in all embodiments of the present invention are the ones, wherein
R¹ is phenyl;
R² is the amino acid side chain of arginine or 2,4-diaminobutyric acid;
R³ is H, phenylmethyl, naphthylmethyl or (1H-indol-3-yl)methyl and R^{3'} is H or (1H-indol-3-yl)ethyl;
R⁴ and R⁵ are, independently of each other H, propyl or octyl;
with the proviso that only one of R³ or R^{3'} is H and the respective other one of R³ and R^{3'} is not H.

Most preferred in all embodiments according to the present invention are the compounds as listed in table 1, respectively the respective trifluoroacetate or mesylate salts thereof:

**Table 1**

| **#** | **Structure** | **Notation based on amino acid sequence*** |
|---|---|---|
| | 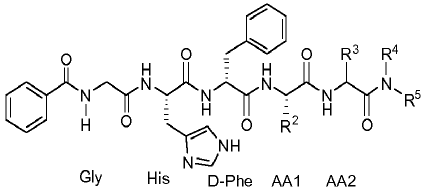 | Bz-Gly-His-DPhe-AA1-AA2-NR⁴R⁵ |
| (I-a) | 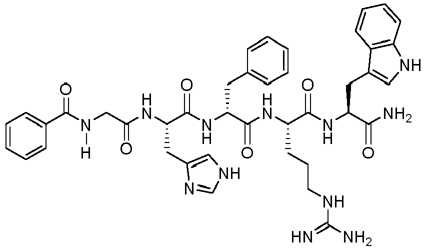 | Bz-Gly-His-D-Phe-Arg-Trp-NH₂ |
| (I-b) | 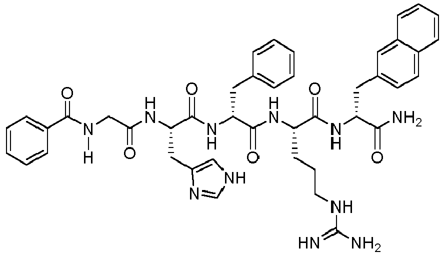 | Bz-Gly-His-D-Phe-Arg-D-2-NaphAla-NH₂ |
| (I-c) | 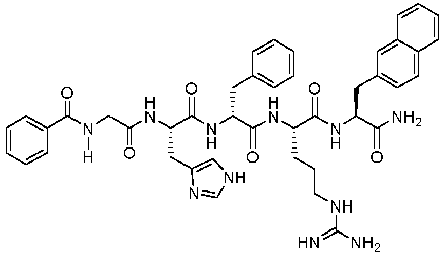 | Bz-Gly-His-D-Phe-Arg-L-2-NaphAla-NH₂ |
| (I-d) | 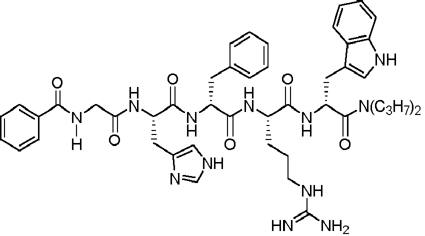 | Bz-Gly-His-D-Phe-Arg-D-Trp-N(Propyl)₂ |
| (I-e) | 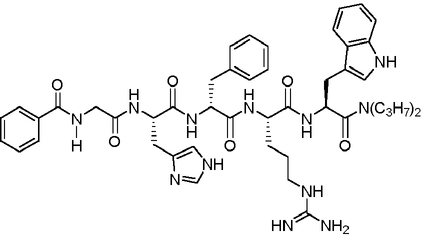 | Bz-Gly-His-D-Phe-Arg-Trp-N(Propyl)₂ |
| (I-f) | 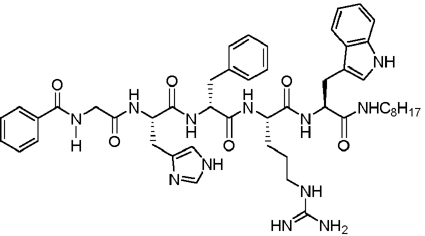 | Bz-Gly-His-D-Phe-Arg-Trp-NHOctyl |
| (I-g) | 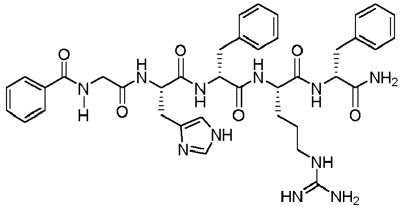 | Bz-Gly-His-D-Phe-Arg-D-Phe-NH₂ |
| (I-h) | 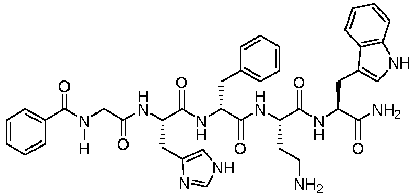 | Bz-Gly-His-D-Phe-Dab-Trp-NH₂ |
| (I-i) | 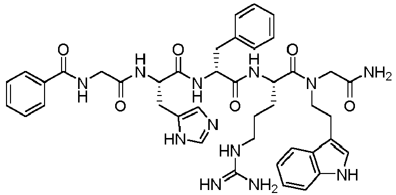 | Bz-Gly-His-D-Phe-Arg-(N-IndEt)Gly-NH₂ *2TFA |

The compounds according to the present invention may be prepared by methods standard in peptide chemistry as e.g. illustrated in WO-2018065345.

Most preferred in all embodiments is Bz-Gly-His-D-Phe-Arg-D-Trp-N(Pr)₂ *2MSA [CAS No. 2210277-15-7], which preferably is used in the form of a solution in glycerin/ water, most preferably in a concentration of about 1000 ppm (w/w).

The compounds of formula (I) respectively a salt thereof are preferably applied to the affected skin area in the form of a topical composition. Preferred topical compositions in all embodiments of the present invention are cosmetic or pharmaceutical compositions further comprising a cosmetically or pharmaceutically acceptable carrier.

The term 'cosmetic composition' as used herein refers to compositions which are used to treat, care for or improve the appearance of the affected skin area. Particular advantageous cosmetic compositions according to the present invention are skin care compositions.

The term 'pharmaceutical composition' as used herein refers to compositions which are used to treat a disorder.

The term 'cosmetically or pharmaceutically acceptable carrier' as used herein refers to a physiologically acceptable medium which is compatible with keratinous substances such as the skin. Suitable carriers are well known in the art and are selected based on the end-use application.

Preferably, the carriers of the present invention are particularly suitable for application to skin (e.g., sunscreens, creams, milks, lotions, masks, serums (e.g. in ethanol or acetone), hydrodispersions, foundations, creams, creamgels, or gels etc.). Such carriers are well-known to one of ordinary skill in the art and can include one or more compatible liquid or solid filler diluent, excipient, additive or vehicle which are suitable for application to skin. The exact amount of carrier will depend upon the level of the compound of formula (I) and any other optional ingredients that one of ordinary skill in the art would classify as distinct from the carrier (e.g., other active components). The compositions of the present invention preferably comprise from about 75% to about 99.999%, more preferably from about 85% to about 99.99%, still more preferably from 90% to about 99%, and most preferably, from about 93% to about 98%, by weight of the composition, of a carrier.

The term 'effective amount' as used herein refers to an amount necessary to obtain the physiological effect. The physiological effect may be achieved by one single dose or by repeated doses. The dosage administered may, of course, vary depending upon known factors, such as the physiological characteristics of the particular composition and its mode and route of administration; the age, health and weight of the recipient; the nature and extent of the symptoms; the kind of concurrent treatment; the frequency of treatment; and the effect desired and can be adjusted by a person skilled in the art.

Preferably, the amount of the compound of formula (I) or a salt thereof in the topical compositions according to the present invention is at least 1 ppm based on the total weight of the topical composition. In all embodiments of the present invention the amount of the compound of formula (I) or a salt thereof is preferably selected in the range of about 0.00001 to 0.5 wt.-%, more preferably in the range of 0.0001 to 0.25 wt.-%, most preferably in the range of 0.0001 to 0.1 wt.-%, based on the total weight of the topical composition.

The topical compositions of the present invention can be formulated into a wide variety of product types, including creams, waxes, pastes, lotions, milks, mousses, gels, oils, tonics, and sprays. Preferably the compounds of formula (I) are formulated into lotions, creams, gels, and tonics. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art. The composition may also be in the form of a shaving preparation or an aftershave product intended for application to the skin after shaving.

If compositions of the present invention are formulated as an aerosol and applied to the skin as a spray-on product, a propellant is added to the composition.

The topical compositions according to the present invention may be in the form of a suspension or dispersion in solvents or fatty substances, or alternatively in the form of an emulsion or micro emulsion (in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the cosmetic composition is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the cosmetic composition.

In one embodiment, the topical compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

In a particular advantageous embodiment, the topical compositions according to the present invention are in the form of O/W emulsions comprising an oily phase dispersed in an aqueous phase in the presence of an O/W emulsifier, preferably in the presence of potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

In another particular advantageous embodiment, the topical compositions according to the present invention are after-shaves or toners in the form of aqueous solution or aqueous gels further containing at least one gelling and/ or thickening agent such as e.g. selected from the group of acrylate copolymers of sodium/lecithin, copolymer of hydroxyethyl acrylate/sodium acryloyldimethyl taurate, isohexadecane, polysorbate 60, xanthan gum, carrageenan and mixtures thereof.

The topical compositions according to the invention in general have a pH in the range of 3 to 14. Generally topical compositions for skin care preferably exhibit a pH in the range of 4 to 8 and most preferably a pH in the range of 4 to 7.5. The pH of depilatory creams, however, generally is selected in the range of > 12. The pH can easily be adjusted as desired with suitable acids, such as e.g. citric acid, or bases, such as sodium hydroxide (e.g. as aqueous solution), triethanolamine (TEA Care), Tromethamine (Trizma Base) and Aminomethyl Propanol (AMP-Ultra PC 2000), according to standard methods in the art.

The topical compositions according to the present invention can be prepared by conventional methods in the art such as e.g. by admixing a compound of formula (I) or a salt thereof with all the definitions and preferences given herein with an appropriate carrier such as a cosmetically or pharmaceutically acceptable carrier. The cosmetic compositions of the invention (including the carrier) may comprise further conventional cosmetic adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

The topical compositions according to the present invention should be topically applied to a selected area of the body from which it is desired to reduce hair growth. For example, the composition can be applied to the face, particularly to the beard area of the face, i.e., the cheek, neck, upper lip, and chin.

The topical composition according to the present invention can also be applied to the legs, groin region (bikini area), arms, torso or armpits. The topical compositions according to the present invention are furthermore particularly suitable for reducing the growth of unwanted hair in women having hirsutism or other conditions. In humans, the composition should be suitably applied once a day, preferably at least twice a day, to achieve a perceivable reduction in hair growth. Perception of reduced hair growth could occur as early as 24 hours or 48 hours (for instance, between normal shaving intervals) following use, but may require several months. Reduction in hair growth is demonstrated when, for example, the rate of hair growth is slowed, the need for removal is reduced, the subject perceives less hair on the treated site, or quantitatively, when the weight of hair removed is reduced.

The amount of the topical composition to be applied to the skin is not critical and can easily be adjusted by a person skilled in the art. Preferably the amount is selected in the range of 0.1 to 3 mg/cm² skin, such as preferably in the range of 0.1 to 2 mg/cm² skin and most preferably in the range of 0.5 to 2 mg/cm² skin.

Preferably, the compound of formula (I) or a salt thereof is applied to the skin in an amount of from 0.001 to 10 micrograms of the compound of formula (I) per square centimeter of skin.

In an advantageous embodiment, the present invention is directed to the use of a topical compositions according to the present invention for delaying hair growth in combination with a short-term or instantaneous method of hair removal. By "in combination with" it is meant that the application of the composition of the invention to the surface of the skin within one hour or less of the hair removal by the other method.

The short-term or instantaneous method of hair removal can be performed by any method well known in the art, including, but not limited to chemical or mechanical depilation such as, shaving (whether using razors, electrical shavers, or clippers or the like), wax depilation, chemical depilation (i.e. dissolution of hair by a depilation agent such as an alkaline thioglycolate), mechanical plucking with an epilator and combinations thereof. Other methods by which hair may be removed include electrology (in which electrical current is applied to hair follicles), and laser hair removal. By short-term or instantaneous it is meant that the hair removal method removes the hair by cutting, dissolution or plucking, but does not prevent or inhibit regrowth of the hair.

The time at which the topically active or the topical composition is applied to the skin, as well as the amount of time for which the composition remains on the skin, may vary, but is suitably within one hour of instantaneous hair removal. Preferably the topical composition is applied to the skin surface either immediately before, immediately after or simultaneously with instantaneous hair removal. More preferably, the topical composition is applied either simultaneously with or immediately following hair removal, most preferably immediately following instantaneous hair removal, and is left on the skin for a period sufficient to obtain an effect to delay hair growth, preferably at least five minutes and more preferably at least fifteen minutes. Most preferably, the composition is allowed to remain on the skin indefinitely to permit absorption into the skin. An advantage of this aspect of the invention is that the compounds of formula (I) or a salt thereof combines a skin tanning effect with the inhibition, delay or reduction in hair growth.

Except where the context requires otherwise, the uses and methods of the invention may be used in the inhibition of hair growth in both human and non-human subjects. For example, the invention may be used in animals which suffer from in-growing hairs or hypertrichosis, especially domestic animals such as cats and dogs. Preferably, however, the uses and methods of the invention are used in the inhibition of hair growth in human subjects.

Inhibition of hair growth may be assessed with reference to the elongation of the hair shaft. It will be appreciated that an agent that reduces elongation of a hair shaft as compared to a suitable control demonstrates the ability to inhibit hair growth.

Suitably the agents, methods, or compositions of the invention may be able to bring at least a 5 % inhibition of hair growth, i.e. a 5 % reduction in the elongation of the hair when compared to a respective control (i.e. non-treated/ vehicle treated area). In a suitable embodiment, an agent, method, or composition of the invention may be able to bring about a 10%, 15% or 20% inhibition of hair growth. The requisite extent of inhibition may be demonstrated in any of the experimental models of hair growth elongation as illustrated in the example.

In a suitable embodiment, the hair growth to be inhibited is regrowth of hair after hair removal. The uses, methods, and compositions of the invention may thus be used to prolong the effects of hair removal. This may allow greater time to elapse between incidences of hair removal. In a suitable embodiment, an agent, method, or composition of the invention may be able to increase the time elapsing between incidences of hair removal by 1, 2, 3, 4, 5, 6, 7, 8, 9 or 10 days or more. The increase in the time between incidences of hair removal achieved may be determined with reference to suitable controls.

In a suitable embodiment the topical composition according to the present invention may be formulated for use in combination with a device which enhances transdermal penetration of the composition. The composition may thus be applied to the area requiring hair growth inhibition prior to use of the device, or concurrently with use of the device. Merely by way of example such devices may include devices which produce electrical current such as galvanic devices, lasers, devices which produce vibrations such as ultrasound, and massage devices which enhance blood circulation around the hair follicles.

The invention is further illustrated with reference to the following, non-limiting examples, in which all percentages are by weight based on total weight unless otherwise specified.

### Experimental part

### 1. Hair growth inhibition

*Microdissection of human hair follicles:* Human scalp skin samples containing mainly anagen VI hair follicles were obtained from elective face-lift plastic surgery. Informed consent was obtained. Hair follicles were truncated below the dermis, while the remaining hair bulb was separated from the dermal/subcutaneous tissue and placed in appropriate culture medium. Before the experiment, each hair follicle underwent a stringent selection, that is operated by controlling the hair shaft elongation following 18 hrs of organ culture, as well as the general organ morphology. Only the hair follicles that grew at least 0.19 ± 0.02 mm/18 hrs were used for the experiment.

*Treatment:* The hair growth inhibition of Bz-Gly-His-D-Phe-Arg-D-Trp-N(Pr)₂ *2MSA in the concentrations as indicated in table 2 was assessed versus control, i.e. versus a treatment with the vehicle (i.e. DMSO) alone.
*Evaluation of hair growth inhibition:* pictures of the hair follicles in culture were taken at baseline and after 4 days and the hair shaft elongation was measured to determine the hair growth.

**Table 2: Results**

| **#** | **Concentration** | **% hair growth inhibition** | **Significance vs vehicle control** (paired t-test) |
|---|---|---|---|
| Inv-1 | 1 nM | 12 | p = 0.067 |
| Inv-2 | 100 nM | 29 | p = 0.019 |

As can be retrieved from the results depicted in table 2, the compounds according to the present invention significantly reduced the hair growth.

### 2. Topical anti-hair growth compositions

Generally, the ingredients are listed by their INCI names, with the exception of the compound of formula (I) which is labeled as Bz-Gly-His-D-Phe-Arg-D-Trp-N(Pr)₂ *2MSA (compound of formula (Id) in table I in the form of its mesylate salt).

| **1. Face Splash** | % w / w |
|---|---|
| BUTYLENE GLYCOL | 2 |
| PANTHENOL | 0.1 |
| ALLANTOIN | 0.15 |
| LACTIC ACID | 1 |
| BETAINE | 0.5 |
| MANNITOL | 1 |
| ALCOHOL | 25 |
| ALOE BARBADENSIS LEAF EXTRACT | 5.0 |
| Bz-Gly-His-D-Phe-Arg-D-Trp-N(Pr)₂ *2MSA | 0.002 |
| GLYCERIN | 2.0 |
| POLYSORBATE-20 | 0.5 |
| PEG-60 HYDROGENATED CASTOR OIL | 0.2 |
| PERFUME | 0.1 |
| AQUA | Ad 100 |

| **2. Moisturizing toner** | % w / w |
|---|---|
| BUTYLENE GLYCOL | 5 |
| CAPRYLYL-GLYCOL | 0.5 |
| HEXYLENE GLYCOL | 1 |
| PROPYLENE GLYCOL | 1 |
| SORBITOL | 1 |
| PANTHENOL | 0.1 |
| LACTIC ACID | 1 |
| SODIUM GLUCONATE | 0.05 |
| BETAINE | 0.5 |
| MANNITOL | 1 |
| ALCOHOL | 3 |
| BIS-PEG-18 METHYL ETHER DIMETHYL SILANE | 0.1 |
| SODIUM HYALURONATE | 0.01 |
| SACCHARIDE ISOMERATE | 0.5 |
| YEAST EXTRACT | 0.1 |
| ALOEALOE BARBADENSIS LEAF EXTRACT | 1 |
| Bz-Gly-His-D-Phe-Arg-D-Trp-N(Pr)₂ *2MSA | 0.001 |
| GLYCERIN | 1 |
| POLYSORBATE-20 | 0.5 |
| PEG-60 HYDROGENATED CASTOR OIL | 0.2 |
| FRAGRANCE, COLOURS | 0.1 |
| WATER | Ad 100 |

| **3. Soothing Gel** | % w / w |
|---|---|
| AQUA | Ad 100 |
| CYCLOPENT ASILOXANE | 5.00 |
| BUTYLENE GLYCOLBUBUbbBBB | 4.00 |
| SORBITOL | 1.00 |
| GLYCERIN | 3.00 |
| ACETYL GLUCOSAMINE | 1.00 |
| DIMETHICONE | 2.00 |
| MENTHOL | 0.1 |
| MENTHA PIPERITA | 0.1 |
| SODIUM HYALURONATE | 0.25 |
| ACRYLATES/C10-30 ALKYL ACRYLATE CROSSPOLYMER | 0.20 |
| AMMONIUM ACRYLOYDIMETHYLTAURATE/VP COPOLYMER | 0.2 |
| ALLANTOIN | 0.20 |
| CAFFEINE | 0.10 |
| Bz-Gly-His-D-Phe-Arg-D-Trp-N(Pr)₂ *2MSA | 0.002 |
| TROMETHAMINE | 0.20 |
| PENTYLENE GLYCOL | 3.00 |
| DISODIUM EDTA | 0.05 |
| PHENOXYETHANOL | 0.50 |
| POTASSIUM SORBATE | 0.15 |
| SODIUM BENZOATE | 0.20 |

| **4. After shave balm** | **% w / w** |
|---|---|
| POTASSIUM CETYL PHOSPHATE | 1.50 |
| ISOHEXADECANE | 4.00 |
| SILICA | 1.00 |
| TOCOPHEROL | 0.05 |
| C12-15 ALKYL BENZOATE | 3.00 |
| ISOPROPYL MYRISTATE | 5.00 |
| CETEARYL ALCOHOL | 2.00 |
| AQUA | Ad 100 |
| GLYCERIN | 2.00 |
| SORBITOL | 2.00 |
| ALCOHOL | 4.00 |
| PHENOXYETHANOL, ETHYLHEXYLGLYCERIN | 1.00 |
| XANTHAN GUM | 0.20 |
| ALLANTOIN | 0.30 |
| Bz-Gly-His-D-Phe-Arg-D-Trp-N(Pr)₂ *2MSA | 0.003 |
| TRITICUM VULGARE GERM EXTRACT, SACCHAROMYCES CEREVISIAE EXTRACT, SODIUM HYALURONATE, PANTHENOL, AQUA, DISODIUM COCOAMPHODIACETATE, PVP, PHENOXYETHANOL, SODIUM METABISULFITE, ETHYLHEXYLGLYCERIN, PANTOLACTONE | 2.00 |
| GLYCERIN, AQUA, CITRIC ACID, SODIUM BENZOATE, POTASSIUM SORBATE, PEUCEDANUM OSTRUTHIUM LEAF EXTRACT | 2.00 |

| **5. After Shave gel** | **% w / w** |
|---|---|
| HYDROXYETHYLCELLULOSE | 1.20 |
| GLYCERIN | 3.00 |
| AQUA | Ad 100 |
| ALOE BARBADENSIS LEAF JUICE | 2.00 |
| AQUA | 20.00 |
| PANTHENOL, AQUA | 1.25 |
| NIACINAMIDE | 5.00 |
| ALCOHOL | 10.00 |
| PEG/PPG-20/6 DIMETHICONE | 0.50 |
| Bz-Gly-His-D-Phe-Arg-D-Trp-N(Pr)₂ *2MSA | 0.003 |
| PEG-40 HYDROGENATED CASTOR OIL | 0.80 |
| PROPYLENE GLYCOL | 5.00 |

| **6. After Shave SPF 15** | **% w / w** |
|---|---|
| AQUA | Ad 100 |
| PROPYLENE GLYCOL | 5.00 |
| BUTYLENE GLYCOL | 5.00 |
| ACRYLATES/C10-30 ALKYL ACRYLATE CROSSPOLYMER | 0.30 |
| DISODIUM EDTA | 0.10 |
| BUTYL METHOXYDIBENZOYLMETHANE | 4.00 |
| OCTOCRYLENE | 4.00 |
| POLYSILICONE-15 | 2.00 |
| ETHYLHEXYL SALICYLATE | 5.00 |
| HOMOSALATE | 8.00 |
| NEOPENTYL GLYCOL DIHEPTANOATE, PROPYLENE GLYCOL DIBENZOATE | 3.00 |
| PHENOXYETHYL CAPRYLATE | 2.00 |
| SILICA | 2.00 |
| ALCOHOL DENAT., AQUA | 10.00 |
| PHENOXYETHANOL, CAPRYLYL GLYCOL | 0.50 |
| Bz-Gly-His-D-Phe-Arg-D-Trp-N(Pr)₂ *2MSA | 0.001 |
| Perfume | 0.15 |
| SODIUM HYDROXIDE | qs. |

| **7. Body Lotion 1** | **% w / w** |
|---|---|
| SORBITAN STEARATE, SUCROSE COCOATE | 2.50 |
| HYDROGENATED COCO-GLYCERIDES | 2.50 |
| GLYCERYL STEARATE | 0.80 |
| MINERAL OIL | 2.00 |
| CAPRYLIC/CAPRIC TRIGLYCERIDE | 10.00 |
| AQUA | Ad 100 |
| GLYCERIN | 4.00 |
| XANTHAN GUM | 0.70 |
| DISODIUM EDTA | 0.10 |
| NIACINAMIDE | 5.00 |
| ACRYLATES/C10-30 ALKYL ACRYLATE CROSSPOLYMER | 0.15 |
| PANTHENOL, AQUA | 0.50 |
| Bz-Gly-His-D-Phe-Arg-D-Trp-N(Pr)₂ *2MSA | 0.0025 |
| PHENOXYETHANOL, ETHYLHEXYLGLYCERIN | 1.00 |
| POTASSIUM HYDROXIDE, AQUA | 0.70 |

| **8. Body Lotion 2** | **% w / w** |
|---|---|
| GLYCERYL STEARATE SE | 2.7 |
| ISOPROPYL PALMITATE | 2.5 |
| GLYCERYL GLUCOSIDE | 1.50 |
| DIMETHICONE | 1.00 |
| CETEARYL ALCOHOL | 2.0 |
| DICAPRYLYL ETHER | 7.00 |
| SODIUM CETEARYL SULFATE | 0.2 |
| AQUA | Ad 100 |
| GLYCERIN | 8.00 |
| XANTHAN GUM | 0.10 |
| DISODIUM EDTA | 0.10 |
| SODIUM CARBOMER | 0.20 |
| PANTHENOL, AQUA | 0.50 |
| Bz-Gly-His-D-Phe-Arg-D-Trp-N(Pr)₂ *2MSA | 0.002 |
| ALCOHOL | 4.00 |
| PHENOXYETHANOL, BENZYL ALCOHOL | 1.00 |
| PARFUM | 0.3 |

| **9. Depilatory Cream 1** | **% w / w** |
|---|---|
| AQUA | Ad 100 |
| GLYCERIN | 3.00 |
| HYDROGENATED POLYDECENE | 7.00 |
| OZOKERITE | 3.00 |
| PHENOXYETHANOL, METHYLPARABEN, ETHYLPARABEN, BUTYLPARABEN, PROPYLPARABEN | 0.50 |
| SODIUM ACRYLATES COPOLYMER, HYDROGENATED POLYISOBUTENE, PHOSPHOLIPIDS, POLYGLYCERYL-10 STEARATE, HELIANTHUS ANNUUS (SUNFLOWER) SEED OIL | 7.00 |
| AQUA, POTASSIUM THIOGLYCOLATE | 10.00 |
| Bz-Gly-His-D-Phe-Arg-D-Trp-N(Pr)₂ *2MSA | 0.003 |
| SODIUM HYDROXIDE | Up to pH>12 |

| **10. Depilatory Cream 2** | **% w / w** |
|---|---|
| AQUA | Ad 100 |
| PROPANEDIOL | 5.00 |
| UREA | 3.00 |
| CETEARETH-12 | 2.00 |
| CETEARYL ALCOHOL, SODIUM CETEARYL SULFATE | 4.00 |
| GLYCERYL COCOATE, GLYCERYL STEARATE, PEG-4, CETEARTH-25, PEG-32, PEG-6, CAPRYLIC/CAPRIC TRIGLYCERIDE, GLYCERYL RICINOLEATE | 2.00 |
| PARAFFINUM LIQUIDUM | 2.00 |
| ISOPROPYL MYRISTATE | 1.00 |
| CALCIUM THIOGLYCOLATE TRIHYDRATE | 7.50 |
| Bz-Gly-His-D-Phe-Arg-D-Trp-N(Pr)₂ *2MSA | 0.003 |
| CALCIUM HYDROXIDE | 1.50 |

| **11. SHAVING FOAM (AEROSOL)** | **% w / w** |
|---|---|
| Blend Composition: | |
| AQUA | Ad 100 |
| PRESERVATIVES | q.s |
| GLYCERINE | 3.0 |
| TRIETHANOLAMINE | 3.50 |
| PEG/PPG-20/6 Dimethicone | 2.00 |
| STEARIC ACID | 7.5 |
| CETYL ALCOHOL | 0.5 |
| GLYCERETH-2 COCOATE | 0.5 |
| COCOAMIDOPROPYL BETAINE 50% | 1.0 |
| COCAMINE OXIDE 30% | 3.0 |
| PERFUME | q.s |
| Bz-Gly-His-D-Phe-Arg-D-Trp-N(Pr)₂ *2MSA | 0.003 |

| *Aerosol prepartion* | |
|---|---|
| Previous Blend | 95 |
| Propellant | 5 |

| **12. SHAVING GEL (AEROSOL)** | **% w / w** |
|---|---|
| Blend Composition: | |
| AQUA | Ad 100 |
| PROPYLENE GLYCOL | 0.5 |
| SORBITOL | 2.0 |
| GLYCERINE | 8.0 |
| TRIETHANOLAMINE | 3.50 |
| ALLANTOIN | 0.1 |
| HYDROXYETHYLCELLULOSE | 0.3 |
| GLYCERYL OLEATE | 2.00 |
| STEARIC ACID | 2.0 |
| PALMITIC ACID | 5.0 |
| MYRISTIC ACID | 1.0 |
| PEG-90m | 0.5 |
| PEG-23Mm | 0.5 |
| PVM/MA Copolymer | 0.2 |
| BHT | 0.05 |
| GLYCERYL ACRYLATE/ACRYLIC ACID COPOLYMER | 0.1 |
| PERFUME | q.s |
| Bz-Gly-His-D-Phe-Arg-D-Trp-N(Pr)₂ *2MSA | 0.003 |

| *Aerosol preparation* | |
|---|---|
| Previous Blend | 90 |
| Propellant | 10 |

## Claims

1. A cosmetic method of reducing hair growth, said method comprising selecting an area of skin from which reduced hair growth is desired and applying to said area a topical composition comprising at least one compound of formula (I) 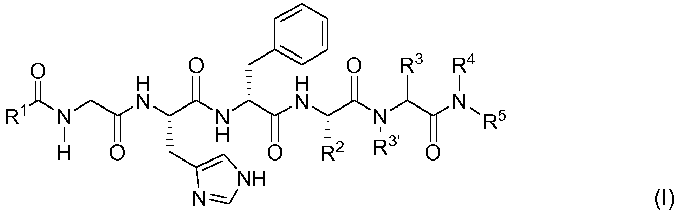 wherein
R¹ is are selected from the group consisting of a C₁-C₁₀ alkyl group, a C₃-C₆ cycloalkyl group, an aryl group or an aryl C₁-C₆ alkyl group;
R² is an amino acid side chain of a basic amino acid or an amino acid side chain of a 2-amino C₂₋₈ alkanoic acid;
R³ and R^{3'} are selected from the group consisting of H, an arylC₁-C₆ alkyl group or a heteroarylC₁-C₆alkyl group, wherein the aryl respectively the heteroaryl can optionally be substituted,
R⁴ and R⁵ are, independently of each other, H or a C₁-C₁₀ alkyl group; and with the proviso that only one of R³ or R^{3'} is H and the respective other one of R³ and R^{3'} is not H
or a salt thereof in an amount effective to reduce hair growth.

2. A topical composition comprising a compound of formula (I) 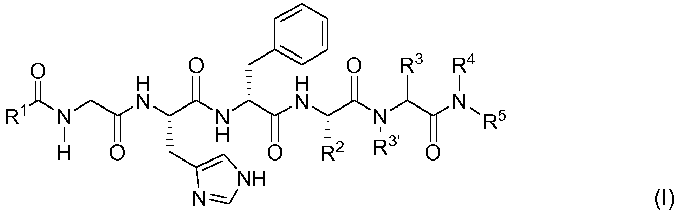 wherein
R¹ is are selected from the group consisting of a C₁-C₁₀ alkyl group, a C₃-C₆ cycloalkyl group, an aryl group or an aryl C₁-C₆ alkyl group;
R² is an amino acid side chain of a basic amino acid or an amino acid side chain of a 2-amino C₂₋₈ alkanoic acid;
R³ and R^{3'} are selected from the group consisting of H, an arylC₁-C₆ alkyl group or a heteroarylC₁-C₆alkyl group, wherein the aryl respectively the heteroaryl can optionally be substituted,
R⁴ and R⁵ are, independently of each other, H or a C₁-C₁₀ alkyl group; and
with the proviso that only one of R³ or R^{3'} is H and the respective other one of R³ and R^{3'} is not H
or a salt thereof for the treatment of individuals suffering from a disorder resulting in excessive hair growth such as hirsutism or hypertrichosis.

3. The method or compound according to anyone of the preceding claims, wherein
R¹ is phenyl;
R² is the amino acid side chain of arginine or 2,4-diaminobutyric acid;
R³ is selected from the group consisting of H, phenylmethyl, naphthylmethyl or (1H-indol-3-yl)methyl and R^{3'} is H or (1H-indol-3-yl)ethyl; and
R⁴ and R⁵ are, independently of each other H, propyl or octyl.

4. The method or compound according to anyone of the preceding claims, wherein the compound of formula (I) is Bz-Gly-His-D-Phe-Arg-Trp-N(Propyl)₂, preferably in the form of its mesylate salt.

5. The method or compound according to anyone of the preceding claims, wherein the amount of the compound of formula (I) or the salt thereof in the composition is selected in the range of about 0.00001 to 0.5 wt.-%, preferably in the range of 0.0001 to 0.25 wt.-%, more preferably in the range of 0.0001 to 0.1 wt.-%, based on the total weight of the composition.

6. The method or compound according to anyone of the preceding claims, wherein the compound of formula (I) or the salt thereof is applied to the skin in an amount of from 0.001 to 10 micrograms of the compound of formula (I) or the salt thereof per square centimeter of skin.

7. The method or the compound according to anyone of the preceding claims, wherein the compound of formula (I) or the salt thereof is provided in an amount sufficient to obtain at least 20% inhibition of hair growth hair.

8. The method or the compound according to anyone of the preceding claims wherein the composition is in the form of an oil-in-water (O/W) emulsion comprising an oily phase dispersed in an aqueous phase in the presence of an O/W emulsifier.

9. The method or the compound according to anyone of the preceding claims wherein the composition is and after-shave or a toner in the form of an aqueous solution or an aqueous gel.

10. The method or compound according to anyone of the preceding claim, wherein the composition is applied to the area of skin in combination with a mechanical or chemical depilation or epilation.

11. The method according to anyone of the preceding claims, wherein the area of the skin is selected from the areas on the face of a human, on a leg of a human, on an arm of the human, in an armpit of the human, in the groin of the human and/ or on the torso the human.

12. Cosmetic use of a compound of formula (I) 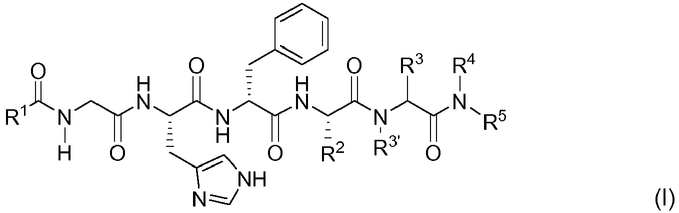 wherein
R¹ is are selected from the group consisting of a C₁-C₁₀ alkyl group, a C₃-C₆ cycloalkyl group, an aryl group or an aryl C₁-C₆ alkyl group;
R² is an amino acid side chain of a basic amino acid or an amino acid side chain of a 2-amino C₂₋₈ alkanoic acid;
R³ and R^{3'} are selected from the group consisting of H, an arylC₁-C₆ alkyl group or a heteroarylC₁-C₆alkyl group, wherein the aryl respectively the heteroaryl can optionally be substituted,
R⁴ and R⁵ are, independently of each other, H or a C₁-C₁₀ alkyl group; and
with the proviso that only one of R³ or R^{3'} is H and the respective other one of R³ and R^{3'} is not H
or a salt thereof for the inhibition of hair growth in an individual in need thereof.

13. The use according to claim 12, wherein the compound of formula (I) or a salt thereof is Bz-Gly-His-D-Phe-Arg-Trp-N(Propyl)₂, preferably in the form of its mesylate salt.
